(19) Europäisches Patentamt
European Patent Office
Office européen des brevets

(11) Veröffentlichungsnummer : **0 022 978**
**B1**

(12) **EUROPÄISCHE PATENTSCHRIFT**

(45) Veröffentlichungstag der Patentschrift : **28.04.82**

(51) Int. Cl.³ : **C 07 C 69/72**, C 07 C 67/46, C 08 F216/14

(21) Anmeldenummer : **80103890.2**

(22) Anmeldetag : **08.07.80**

(54) **Acetylacetoxyalkyl-allyl-ether, ihre Herstellung und Verwendung.**

(30) Priorität : **11.07.79 DE 2927933**

(43) Veröffentlichungstag der Anmeldung : **28.01.81 (Patentblatt 81/04)**

(45) Bekanntmachung des Hinweises auf die Patenterteilung : **28.04.82 Patentblatt 82/17**

(84) Benannte Vertragsstaaten : **AT BE CH DE FR GB IT LI NL SE**

(56) Entgegenhaltungen :
**DE - A - 2 626 173**
**DE - A - 2 628 760**
**DE - B - 2 117 571**

(73) Patentinhaber : **HOECHST AKTIENGESELLSCHAFT**
**Postfach 80 03 20**
**D-6230 Frankfurt/Main 80 (DE)**

(72) Erfinder : **Braun, Helmut, Dr.**
**Königsbergerstrasse 74**
**D-6239 Kriftel (DE)**
Erfinder : **Rinno, Helmut, Dr.**
**Goethestrasse 39**
**D-6238 Hofheim am Taunus (DE)**
Erfinder : **Rauterkus, Karl Josef, Dr.**
**Im Hain 2**
**D-6233 Kelkheim (Taunus) (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Imprimerie Jouve, 18, rue St-Denis, 75001 Paris, France

EP 0 022 978 B1

# Acetylacetoxyalkyl-allyl-ether, ihre Herstellung und Verwendung

Die Erfindung bezieht sich auf Acetylacetoxyalkyl-allylether, ihre Herstellung und Verwendung.

Es ist bekannt, daß ungesättigte Acetessigsäureester sich als Comonomere bei der Polymerisation bestimmter ungesättigter Verbindungen eignen (vgl. z.B. deutsche Offenlegungsschriften 25 35 372, 25 35 374 und 26 28 760). Dabei wird die Copolymerisation in einem wäßrigen Medium durchgeführt, so daß eine wäßrige Kunststoff-Dispersion resultiert. Diese Kunststoff-Dispersionen werden als Bindemittel-Dispersionen in Anstrichfarben verwendet (vgl. z.B. Deutsche Offenlegungsschrift 25 35 373).

Aufgabe der Erfindung ist die Bereitstellung von Verbindungen, die eine olefinisch ungesättigte Doppelbindung und mindestens eine Acetoacetylgruppe aufweisen, ohne großen technischen Aufwand zugänglich sind und sich mit einer Vielzahl von ungesättigten Verbindungen copolymerisieren lassen.

Die Erfindung betrifft nun Acetylacetoxyalkyl-allyl-ether der Formel (I)

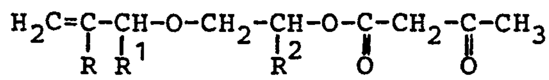

oder der Formel (II)

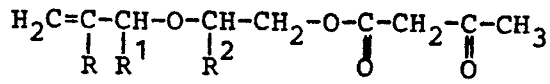

und deren Gemische,

wobei R ein Wasserstoffatom oder einen Methylrest, $R^1$ ein Wasserstoffatom oder einen Alkylrest mit 1, 2 oder 3 Kohlenstoffatomen und $R^2$ ein Wasserstoffatom oder einen — gegebenenfalls ein oder mehrere Sauerstoffatome oder ein Halogenatom enthaltenden — Kohlenwasserstoffrest mit 1 bis 8 Kohlenstoffatomen bedeuten. Der Rest $R^1$ bedeutet vorzugsweise ein Wasserstoffatom, während der Rest $R^2$ vorzugsweise (a) ein Wasserstoffatom, (b) ein Alkylrest mit 1, 2 oder 3 Kohlenstoffatomen, der ein Halogenatom, einen Hydroxylrest, einen Acyloxyrest mit 3 bis 6 Kohlenstoffatomen oder eine Acetylacetoxygruppe enthalten kann, oder (c) ein Arylrest mit 6, 7 oder 8 Kohlenstoffatomen ist.

Die Erfindung betrifft somit insbesondere Acetylacetoxy-alkyl-allyl-ether der Formel (III)

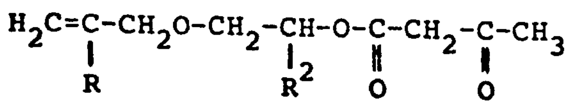

oder der Formel (IV)

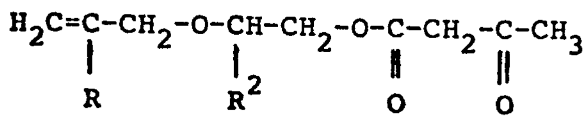

und deren Gemische,

wobei R ein Wasserstoffatom oder einen Methylrest und $R^2$ (a) ein Wasserstoffatom, (b) einen Alkylrest mit 1, 2 oder 3 Kohlenstoffatomen, der mit einem Halogenatom, vorzugsweise einem Chloratom, einem Hydroxylrest, einem Acyloxyrest mit 3 bis 6 Kohlenstoffatomen oder einer Acetylacetoxygruppe substituiert sein kann, oder (c) einen Arylrest mit 6, 7 oder 8 Kohlenstoffatomen, vorzugsweise einen Phenylrest, bedeuten.

Erfindungsgemäße Acetylacetoxyalkyl-allyl-ether sind beispielsweise [2-(Acetylacetoxy)-ethyl]-allyl-ether, [2-(Acetylacetoxy)-ethyl]-methallyl-ether, [2-(Acetylacetoxy)-ethyl]-1-methylallyl-ether, [2-(Acetylacetoxy)-ethyl]-1-ethylallyl-ether, [2-(Acetylacetoxy)-ethyl]-1-propylallyl-ether, [2-(Acetylacetoxy)-propyl]-allyl-ether, [2-(Acetylacetoxy)-propyl]-methallyl-ether, [2-(Acetylacetoxy)-propyl]-1-methylallyl-ether sowie [2-(Acetylacetoxy)-2-chlormethyl]-ethyl-allyl-ether, [2-(Acetylacetoxy)-2-hydroxy-methyl]-ethyl-allyl-ether, [2-Acetylacetoxy)-2-phenyl]-ethyl-allyl-ether, [2-(Acetylacetoxy)-2-p-tolyl]-ethyl-allyl-ether, [2,3-Bis(acetylacetoxy)-propyl]-allyl-ether, (2-Acetylacetoxy-3-acryloyloxy-propyl)-allyl-ether, (2-Acetylacetoxy-3-methacryloyloxy-propyl)-allyl-ether und die entsprechenden Methallylether.

Die Herstellung der erfindungsgemäßen Verbindungen ist auf verschiedenen Wegen möglich. Beispielsweise kann eine Williamson-Synthese durchgeführt werden (a) mit einem Alkalisalz eines — gegebenenfalls substituierten — (2-Hydroxyethyl)-acetessigsäureesters und einem — gegebenenfalls substituierten — Allylhalogenid oder (b) in umgekehrter Weise mit einem — gegebenenfalls substituierten — (2-Halogenethyl)-acetessigsäureester und einem — gegebenenfalls substituierten-Alkali-allylalkoholat. Ferner kann man in analoger Weise zunächst einen — gegebenenfalls substituierten — Allyl-glykol-ether herstellen, diesen in den Allyl-glykol-essigsäureester überführen und letzteren dann mittels einer Claisen-Kondensation mit Acetessigsäureethylester umsetzen.

Auf besonders einfache und bevorzugte Weise sind die erfindungsgemäßen Verbindungen dadurch herzustellen, daß man zunächst einen — gegebenenfalls substituierten — Allylalkohol mit einem Epoxid und den erhaltenen Hydroxy-alkyl-allyl-ether dann mit Diketen umsetzt. Dabei wird unter üblichen Bedingungen vorzugsweise zunächst ein Alkohol der Formel (V)

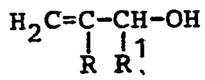

in der R und $R^1$ die bei Formel (I) genannten Bedeutungen haben, mit einem Epoxid der Formel (VI)

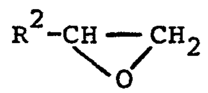

umgesetzt, in der $R^2$ die bei Formel (I) genannte Bedeutung hat, und der erhaltene Hydroxy-alkyl-allyl-ether dann mit Diketen umgesetzt wird.

Als Alkohol wird vorzugsweise Allylalkohol oder Methallylalkohol verwendet ; weitere Beispiele sind 1-Methylallyl-alkohol, 1-Ethylallylalkohol, 1-Propylallylalkohol und 1,2-Dimethylallylalkohol.

Besonders geeignete Epoxide sind solche der Formel (VI), in der $R^2$ ein Wasserstoffatom, einen Alkylrest mit 1, 2 oder 3 Kohlenstoffatomen, der mit einem Halogenatom, vorzugsweise einem Chloratom, einen Hydroxylrest oder einem Acylrest mit 3 bis 6, vorzugsweise 3 oder 4 Kohlenstoffatomen, substituiert sein kann, oder einen Arylrest mit 6, 7 oder 8 Kohlenstoffatomen, vorzugsweise einen Phenylrest, bedeutet. Als Beispiele sind vor allem zu nennen Ethylenoxid, Propylenoxid, Glycid, Epichlorhydrin, Styroloxid und Glycidylester wie Glycidylacrylat, Glycidylmethacrylat und Glycidylcrotonat.

Die Reaktion zwischen dem Alkanol und dem Epoxid wird unter üblichen Bedingungen, ggf. in Gegenwart eines inerten Lösungsmittels, jedoch vorzugsweise in Substanz, durchgeführt. Die Reaktionstemperatur beträgt dabei 0 bis 120 °C, vorzugsweise 20 bis 100 °C. Üblicherweise wird bei Normaldruck gearbeitet ; ist jedoch ein Reaktionspartner bei der jeweiligen Reaktionstemperatur gasförmig, so kann die Umsetzung auch bei erhöhtem Druck durchgeführt werden. Die Reaktion wird in Abwesenheit eines Katalysators oder vorzugsweise in Gegenwart eines Katalysators durchgeführt, der dann in einer Menge von 0,01 bis 2 Gewichtsprozent, vorzugsweise 0,02 bis 0,5 Gewichtsprozent (bezogen auf die Gesamtmenge der Reaktanten) eingesetzt wird. Als Katalysator eignen sich vor allem stark alkalisch reagierende Substanzen, insbesondere (a) Alkalimetalle, z.B. Natrium, Kalium und Lithium, (b) Alkalialkoholate, vorzugsweise mit 1 bis 4 Kohlenstoffatomen, z.B. Natriummethylat, Natriumethylat, Natriumpropylat, Natrium-t-butylat und die analogen Kaliumverbindungen, sowie insbesondere die Alkalialkoholate der jeweils verwendeten ungesättigten Alkohole, z.B. Natriumallylalkoholat und Natriummethallylalkoholat, und (c) aliphatische Amine, vorzugsweise Trialkylamine mit 3 bis 9 Kohlenstoffatomen, z.B. Trimethylamin, Triethylamin, Triethanolamin, und cyclische Amine, z.B. Pyridin, Piperidin, Morpholin und Piperazin. Ebenfalls geeignet sind sauer reagierende Verbindungen, insbesondere anorganische Säuren, z.B. Salzsäure, Schwefelsäure, Salpetersäure, Phosphorsäure, sowie Lewis-Säuren, z.B. Bortrifluorid und Phosphortrichlorid, die auch in Form ihrer Additionsverbindungen eingesetzt werden können, z.B. als Etherate.

Die Umsetzung des in der ersten Reaktionsstufe erhaltenen Hydroxyalkyl-allyl-ethers, dessen Alkylrest 2 bis 10 und vorzugsweise 2 bis 8 Kohlenstoffatome und dessen Allylrest 3 bis 7 und vorzugsweise 3 oder 4 Kohlenstoffatome aufweist, mit Diketen wird ebenfalls unter üblichen Bedingungen, gegebenenfalls in Gegenwart eines inerten Lösungsmittels, jedoch vorzugsweise in Substanz, durchgeführt, wobei die Reaktionstemperatur im Bereich von 0 bis 120 °C, vorzugsweise 20 bis 100 °C, liegt. Die Umsetzung erfolgt in der Regel bei Normaldruck ; es kann aber auch erhöhter Druck angewandt werden. Empfehlenswert ist die Durchführung der Reaktion in Gegenwart eines Katalysators, der in einer Menge von 0,01 bis 2 Gewichtsprozent, vorzugsweise 0,02 bis 0,5 Gewichtsprozent eingesetzt wird (bezogen auf die Gesamtmenge der Reaktanten). Als Katalysator dienen hier (a) Säuren, (b) saure Salze, (c) Basen oder (d) basische Salze, z.B. Schwefelsäure, Phosphorsäure, p-Toluolsulfonsäure, Natriumhydrogensulfat, Triethylamin, Triethanolamin und Trimethylammoniumacetat.

Die erfindungsgemäßen Acetylacetoxyalkyl-allyl-ether fallen nach den oben beschriebenen Verfahren im allgemeinen in Form von Gemischen aus Verbindungen der Formel (I) und Verbindungen der Formel (II) an ; dies ist für die Anwendung der erfindungsgemäßen Stoffe jedoch ohne Bedeutung. Sie sind vor allem anwendbar als Ligandbildner für Schwermetallionen sowie als Comonomere bei der Polymerisation von Vinylverbindungen wie Vinylester, Acrylsäureester, Olefine, Vinylhalogenide und Vinylaromaten. Bevorzugt ist der Einsatz der Acetylacetoxyalkyl-allyl-ether bei der Emulsionspolymerisation der genannten Vinylverbindungen.

Die nachstehenden Beispiele erläutern die Erfindung. Die Struktur der erfindungsgemäßen Verbindungen wurde jeweils infrarotspektrografisch nachgewiesen. Prozentangaben beziehen sich jeweils auf das Gewicht.

Beispiel 1

a) 58,1 g (1 mol) Allylalkohol werden in einem Vierhalskolben, der mit Rührer, Thermometer, Tropftrichter und Rückflußkühler versehen ist, mit 200 mg blankem metallischen Natrium versetzt und dann zum Sieden (97 °C) erhitzt. Nach Auflösung des Natriums werden dem siedenden Allylalkohol im Laufe von 2 h unter Rühren 61 g (1,05 mol) Propylenoxid gleichmäßig zugegeben. Nach einer anschließenden Nachreaktionszeit von 1 h bei 97 °C wird das Reaktionsgemisch bei einem Druck von 2 mbar destilliert. Es werden 65 g (55 % der Theorie) 2-Hydroxypropyl-(1)-allyl-ether mit einem Siedepunkt von 67 °C bei 2 mbar und einem Brechungsindex $n_D^{20} = 1,435\,5$ erhalten.

b) In dem oben beschriebenen Reaktionsgefäß werden 58,1 g (0,5 mol) 2-Hydroxypropyl-(1)-allyl-ether mit 0,03 g Trimethylammoniumacetat versetzt und auf eine Temperatur von 75 °C erhitzt. Unter Einhaltung dieser Temperatur werden dann unter Rühren im Laufe von 30 min 42 g (0,5 mol) Diketen zu der Vorlage getropft. Nach einer anschließenden Nachreaktionszeit von 1 h bei 75 °C wird das Reaktionsgemisch auf Raumtemperatur abgekühlt. Es werden 100 g 2-Acetylacetoxypropyl-(1)-allyl-ether als leicht gelbe Flüssigkeit mit einem Brechungsindex $n_D^{20} = 1,445\,0$ erhalten.

Beispiel 2

In einem Vierhalskolben, der mit Rührer, Thermometer, Tropftrichter und Rückflußkühler versehen ist, wird ein Gemisch aus 1 320 g (10 mol) 2,3-Dihydroxypropyl-(1)-allyl-ether und 9 g metallischem Natrium auf eine Temperatur von 70 °C erhitzt, wobei sich das Natrium auflöst, und unter Einhaltung dieser Temperatur unter Rühren im Laufe von 2 h gleichmäßig mit 1 680 g (20 mol) Diketen versetzt. Nach einer anschließenden Nachreaktionszeit von 1 h bei 80 °C wird das Reaktionsgemisch auf Raumtemperatur abgekühlt. Es werden 3 000 g 2,3-Bis(acetylacetoxy)-propyl-(1)-allyl-ether als gelbe, ölige Flüssigkeit mit einem Brechungsindex $n_D^{20} = 1,462\,5$ erhalten.

Beispiel 3

a) In einem mit Rückflußkühler, Thermometer und Tropftrichter versehenen Reaktionsgefäß wird ein Gemisch aus 87 g (1,5 mol) Allylalkohol und 1 ml Bortrifluorid-Diethyletherat auf eine Temperatur von 60 °C erhitzt. Zu diesem Gemisch werden im Laufe von 45 min 46,25 g (0,5 mol) Epichlorhydrin gleichmäßig zugegeben. Die Reaktion verläuft exotherm. Anschließend wird das Reaktionsgemisch über eine 40-cm-Silbermantelkolonne rektifiziert, wobei zunächst bei einer Temperatur von 24 °C und einem Druck von 15 mbar 55 g nicht umgesetzter Allylalkohol abdestilliert werden. Durch anschliessende Destillation bei 0,08 mbar werden 59,7 g (79,3 % der Theorie) (3-Chlor-2-hydroxy-propyl)-allyl-ether mit einem Siedepunkt von 57 °C und einem Brechungsindex von $n_D^{20} = 1,463\,0$ erhalten.

b) In einem mit Rührer, Thermometer, Rückflußkühler und Tropftrichter versehenen Vierhalskolben wird ein Gemisch aus 37,6 g (0,25 mol) (3-Chlor-2-hydroxy-propyl)-allyl-ether und 0,018 g (0,03 %) Trimethylammoniumacetat auf eine Temperatur von 75 °C erhitzt. Zu diesem Gemisch werden im Laufe von 20 min 21 g (0,25 mol) Diketen gleichmäßig zugetropft. Die Reaktion verläuft exotherm. Es werden 58 g (3-Chlor-2-acetylacetoxy-propyl)-allyl-ether als schwach gelbbraun gefärbte Flüssigkeit mit einem Brechungsindex $n_D^{20} = 1,462\,5$ erhalten.

Beispiel 4

a) In einem mit Rückflußkühler, Thermometer und Tropftrichter versehenen Reaktionsgefäß werden 0,2 g metallisches Natrium in 36 g (0,5 mol) Buten-(1)-ol-(3) (= 1-Methyl-allylalkohol) aufgelöst, und diese Lösung wird auf eine Temperatur von 60 °C erhitzt. Dann werden im Laufe von 2,5 h 30,5 g (0,525 mol) Propylenoxid gleichmäßig zugefügt, und das Reaktionsgemisch wird noch weitere 2,5 h auf einer Temperatur von 90 °C gehalten. Nach Abkühlen auf Raumtemperatur wird das Gemisch über eine 40-cm-Silbermantelkolonne rektifiziert ; dabei werden zunächst bei einer Temperatur von 35 °C und Normaldruck 15 g nicht umgesetztes Propylenoxid und bei einer Temperatur von 23 °C und einem Druck von 27 mbar 19,6 g nicht umgesetztes Butenol abdestilliert. Durch weitere Destillation bei 27 mbar werden 25,1 g (39,1 % der Theorie) (2-Hydroxypropyl)-1-methylallyl-ether mit einem Siedepunkt von 62 °C und einem Brechungsindex $n_D^{20} = 1,426\,0$ erhalten.

b) In einem mit Rührer, Rückflußkühler, Thermometer und Tropftrichter versehenen Vierhalskolben wird ein Gemisch aus 26 g (0,2 mol) (2-Hydroxypropyl)-1-methyl-allyl-ether und 0,013 g (0,03 %) Trimethylammonium-acetat auf eine Temperatur von 75 °C erhitzt. Zu diesem Gemisch werden im Laufe von 30 min 16,8 g (0,2 mol) Diketen gleichmäßig zugetropft. Die Reaktion verläuft exotherm. Es werden 43 g (2-Acetylacetoxypropyl)-methylallyl-ether als schwach bräunlich gefärbte Flüssigkeit mit einem Brechungsindex $n_D^{20} = 1,439\,0$ erhalten.

Beispiel 5

a) In einem mit Rückflußkühler, Thermometer und Tropftrichter versehenen Reaktionsgefäß wird ein

Gemisch aus 87 g (1,5 mol) Allylalkohol und 1 ml Bortrifluorid-Diethyletherat auf eine Temperatur von 55 °C erhitzt. Zu diesem Gemisch werden im Laufe von 80 min 71 g (0,5 mol) Methacrylsäureglycidylester gleichmäßig zugegeben. Die Reaktion verläuft exotherm. Anschließend wird das Reaktionsgemisch über eine 40-cm-Silbermantelkolonne rektifiziert, wobei zunächst bei einer Temperatur von 25 °C und einem Druck von 16 mbar 57 g nicht umgesetzter Allylalkohol abdestilliert werden. Durch anschließende Destillation bei 0,3 mbar werden 61,8 g (61,8 % der Theorie) (3-Methacryloyloxy-propyl)-allyl-ether mit einem Siedepunkt von 99 °C und einem Brechungsindex $n_D^{20}$ = 1,412 0 erhalten.

b) In einem mit Rührer, Rückflußkühler, Thermometer und Tropftrichter versehenen Reaktionsgefäß wird ein Gemisch aus 12 g (0,06 mol) (3-Methacryloyloxy-propyl)-allyl-ether und 0,005 g (0,03 %) Trimethylammoniumacetat auf eine Temperatur von 75 °C erhitzt. Zu diesem Gemisch werden im Laufe von 15 min 5,04 g (0,06 mol) Diketen gleichmäßig zugetropft. Die Reaktion verläuft exotherm. Nach einer anschließenden Nachreaktionszeit von 1 h bei 80 °C wird das Gemisch auf Raumtemperatur abgekühlt. Es werden 17 g (2-Acetylacetoxy-3-methacryloyloxy-propyl)-allyl-ether als schwach gelbbraun gefärbte Flüssigkeit mit einem Brechungsindex $n_D^{20}$ = 1,425 5 erhalten.

Beispiel 6

a) In einem Vierhalskolben, der mit Rührer, Rückflußkühler, Thermometer und Tropftrichter versehen ist, wird eine Lösung aus 54 g (0,87 mol) Ethylenglykol und 8 ml absolutem Toluol im Laufe von 3 h portionsweise mit 4,6 g (0,2 mol) metallischem Natrium versetzt. Danach wird das Gemisch bis zum Schmelzen des Natriums erwärmt und noch 6 h lang bis zur vollständigen Auflösung des Natriums gerührt. Nach Dekantieren des Toluols wird der Kolbeninhalt zweimal mit je 50 ml absolutem Diethylether gewaschen. Das verbleibende Natriumglykolat/Glykol-Gemisch wird auf eine Temperatur von 72 °C erhitzt, und im Laufe von 1 h werden 24,4 g (0,2 mol) Allylbromid zudosiert. Nach einer anschliessenden Nachreaktionszeit von 1 h bei 110 °C wird das Reaktionsgemisch über eine 40-cm-Silbermantelkolonne rektifiziert. Es werden 14 g (68,6 % der Theorie) 2-Hydroxyethyl-allyl-ether mit einem Siedepunkt von 63 °C bei 24 mbar und einem Brechungsindex $n_D^{20}$ = 1,437 5 erhalten.

b) In einem mit Rührer, Thermometer, Rückflüßkuhler und Tropftrichter versehenen Vierhalskolben wird ein Gemisch aus 12 g (0,12 mol) 2-Hydroxyethyl-allyl-ether und 0,006 5 g (0,03 %) Trimethylammoniumacetat auf eine Temperatur von 75 °C erhitzt. Zu diesem Gemisch werden im Laufe von 20 min 9,88 g (0,12 mol) Diketen gleichmäßig zugetropft, und das Gemisch wird dann noch 1 h auf einer Temperatur von 80 °C gehalten (exotherme Reaktion). Es werden 22 g (2-Acetylacetoxy-ethyl)-allyl-ether als schwach gelbbraun gefärbte Flüssigkeit mit einem Brechungsindex $n_D^{20}$ = 1,447 5 erhalten.

**Ansprüche** (für die Vertragsstaaten : BE, CH, LI, DE, FR, GB, IT, NL, SE)

1. Acetylacetoxyalkyl-allyl-ether der Formel (I)

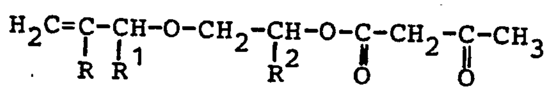

oder der Formel (II)

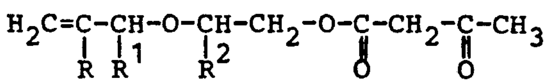

und deren Gemische.
wobei R ein Wasserstoffatom oder einen Methylrest, $R^1$ ein Wasserstoffatom oder einen Alkylrest mit 1, 2 oder 3 Kohlenstoffatomen und $R^2$ ein Wasserstoffatom oder einen — gegebenenfalls ein oder mehrere Sauerstoffatome oder ein Halogenatom enthaltenden — Kohlenwasserstoffrest mit 1 bis 8 Kohlenstoffatomen bedeuten.

2. Acetylacetoxyalkyl-allyl-ether der Formel (III)

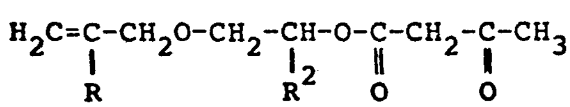

oder der Formel (IV)

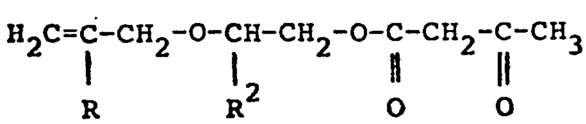

und deren Gemische, wobei R ein Wasserstoffatom oder einen Methylrest und $R^2$ (a) ein Wasserstoffatom, (b) einen Alkylrest mit 1, 2 oder 3 Kohlenstoffatomen, der mit einem Halogenatom, einem Hydroxylrest, einem Acyloxyrest mit 3 bis 6 Kohlenstoffatomen oder einer Acetylacetoxygruppe substituiert sein kann, oder (c) einen Arylrest mit 6,7 oder 8 Kohlenstoffatomen bedeuten.

3. Verfahren zur Herstellung eines Acetylacetoxyalkyl-allyl-ethers nach Anspruch 1, dadurch gekennzeichnet, daß ein Hydroxyalkyl-allyl-ether mit 2 bis 10 Kohlenstoffatomen in der Alkylgruppe und 3 bis 7 Kohlenstoffatomen in der Allylgruppe unter üblichen Bedingungen mit Diketen umgesetzt wird.

4. Verfahren zur Herstellung eines Acetylacetoxyalkyl-allyl-ethers, dadurch gekennzeichnet, daß unter üblichen Bedingungen zunächst ein Alkohol der Formel (V)

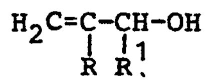

in der R und $R^1$ die bei Formel (I) genannten Bedeutungen haben, mit einem Epoxid der Formel (VI)

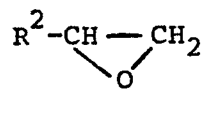

in der $R^2$ die bei Formel (I) genannte Bedeutung hat, und der erhaltene Hydroxyalkyl-allyl-ether dann mit Diketen umgesetzt wird.

5. Verfahren nach Anspruch 4, dadurch gekennzeichnet, daß als Alkohol der Formel (V) Allylalkohol oder Methallylalkohol verwendet wird.

6. Verfahren nach Anspruch 4, dadurch gekennzeichnet, daß als Epoxid ein Epoxid der Formel (VI) verwendet wird, in der $R^2$ ein Wasserstoffatom, einen Alkylrest mit 1, 2 oder 3 Kohlenstoffatomen, der mit einem Halogenatom, einem Hydroxylrest oder einem Acyloxyrest mit 3 bis 6 Kohlenstoffatomen substituiert sein kann, oder einen Arylrest mit 6,7 oder 8 Kohlenstoffatomen bedeutet.

7. Verfahren nach Anspruch 4, dadurch gekennzeichnet, daß die Umsetzungen in Gegenwart eines Katalysators durchgefüíhrt werden.

8. Verwendung eines Acetylacetoxyalkyl-allyl-ethers gemäß Anspruch 1 als Comonomer bei der Polymerisation von Vinylverbindungen.

**Ansprüche** (für den Vertragsstaat AT)

1. Verfahren zur Herstellung eines Acetylacetoxyalkylallylethers, dadurch gekennzeichnet, daß ein Hydroxy-alkylallyl-ether mit 2 bis 10 Kohlenstoffatomen in der Alkylgruppe und 3 bis 7 Kohlenstoffatomen in der Allylgruppe unter üblichen Bedingungen mit Diketen zu einer Verbindung der Formel (I)

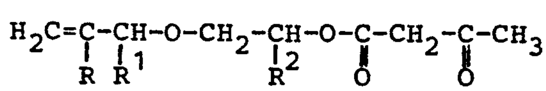

oder der Formel (II)

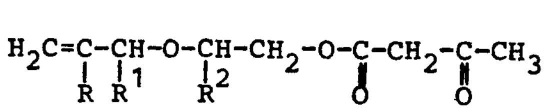

oder zu einem Gemisch von Verbindungen der formel (I) und (II) umgesetzt wird, wobei R ein Wasserstoffatom oder einen Methylrest, $R^1$ ein Wasserstoffatom oder einen Alkylrest mit 1, 2 oder 3 Kohlenstoffatomen und $R^2$ ein Wasserstoffatom oder einen — gegebenenfalls ein oder mehrere Sauerstoffatome oder ein Halogenatom enthaltenden — Kohlenwasserstoffrest mit 1 bis 8 Kohlenstoffatomen bedeuten.

2. Verfahren zur Herstellung eines Acetylacetoxyalkyl-allyl-ethers, dadurch gekennzeichnet, daß unter üblichen Bedingungen zunächst ein Alkohol der Formel (V)

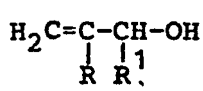

in der R ein Wasserstoffatom oder einen Methylrest und $R^1$ ein Wasserstoffatom oder einen Alkylrest mit 1, 2 oder 3 Kohlenstoffatomen bedeuten, mit einem Epoxid der Formel (VI)

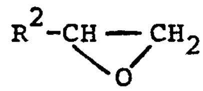

in der $R^2$ ein Wasserstoffatom oder einen — gegebenenfalls ein oder mehrere Sauerstoffatome oder ein Halogenatom enthaltenden — Kohlenwasserstoffrest mit 1 bis 8 Kohlenstoffatomen bedeuten, und der erhaltene Hydroxy-alkyl-allyl-ether dann mit Diketen umgesetzt wird.

3. Verfahren nach Anspruch 2, dadurch gekennzeichnet, daß als Alkohol der Formel (V) Allylalkohol oder Methallyl-alkohol verwendet wird.

4. Verfahren nach Anspruch 2, dadurch gekennzeichnet, daß als Epoxid ein Epoxid der Formel (VI) verwendet wird, in der $R^2$ ein Wasserstoffatom, einen Alkylrest mit 1, 2 oder 3 Kohlenstoffatomen, der mit einem Halogenatom, einem Hydroxylrest oder einem Acyloxyrest mit 3 bis 6 Kohlenstoffatomen substituiert sein kann, oder einen Arylrest mit 6, 7 oder 8 Kohlenstoffatomen bedeutet.

5. Verfahren nach Anspruch 2, dadurch gekennzeichnet, daß die Umsetzungen in Gegenwart eines Katalysators durchgeführt werden.

6. Verfahren zur Herstellung eines ungesättigten Acetessigsäureesters, dadurch gekennzeichnet, daß

a) mit einem Alkalisalz eines — gegebenenfalls substituierten — (2-Hydroxyethyl)-acetessigsäureesters und einem — gegebenenfalls substituierten — Allyl-halogenid eine Williamson-Synthese durchgeführt wird, oder

b) mit einem — gegebenenfalls substituierten — (2-Halogenethyl)-acetessigsäureester und einem — gegebenenfalls substituierten — Alkali-allyl-alkoholat eine Williamson-Synthese durchgeführt wird, oder

c) ein — gegebenenfalls substituierter — Allyl-glykol-ether in einen Allyl-glykol-essigsäureester überführt und letzterer mittels einer Claisen-Kondensation mit Acetessigsäureethylester umgesetzt wird, wodurch ein Acetylacetoxyalkyl-allyl-ether der Formel (I)

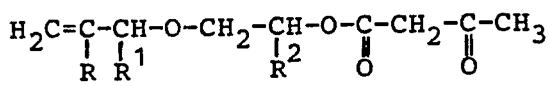

oder der Formel (II)

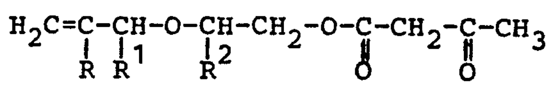

oder deren Gemisch erhalten wird, wobei in den vorgenannten Formeln R ein Wasserstoffatom oder einen Methylrest, $R^1$ ein Wasserstoffatom oder einen Alkylrest mit 1, 2 oder 3 Kohlenstoffatomen und $R^2$ ein Wasserstoffatom oder einen — gegebenenfalls ein oder mehrere Sauerstoffatome oder ein Halogenatom enthaltenden — Kohlenwasserstoffrest mit 1 bis 8 Kohlenstoffatomen bedeuten.

7. Verwendung eines gemäß Anspruch 1 oder 6 herstellbaren Acetylacetoxyalkyl-allyl-ethers als Comonomer bei der Polymerisation von Vinylverbindungen.

**Claims** (for the contracting States : BE, CH, LI, DE, FR, GB, IT, NL, SE)

1. Acetylacetoxyalkyl-allyl ether of formula (I)

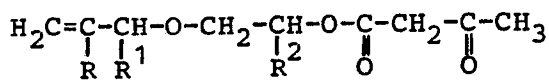

or of formula (II)

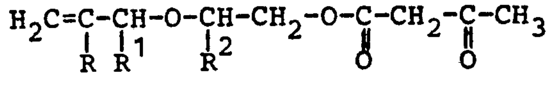

and mixtures thereof,

in which formulae R is a hydrogen atom or a methyl group, $R^1$ is a hydrogen atom or an alkyl group having 1, 2 or 3 carbon atoms and $R^2$ is a hydrogen atom or a hydrocarbon radical having from 1 to 8 carbon atoms and optionally containing one or more oxygen atoms or a halogen atom.

2. Acetylacetoxyalkyl-allyl ether of formula (III)

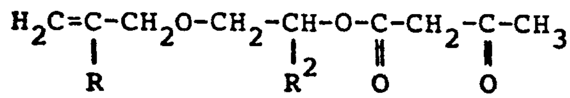

or of formula (IV)

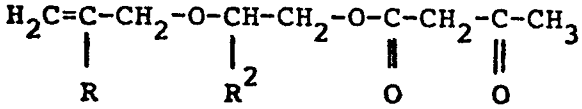

and mixtures thereof,
in which formulae R is a hydrogen atom or a methyl group and $R^2$ is (a) a hydrogen atom, (b) an alkyl group having 1, 2 or 3 carbon atoms, which may be substituted by a halogen atom, a hydroxyl radical, an acyloxy radical having 3 to 6 carbon atoms, or an acetylacetoxy group, or (c) an aryl group having 6, 7 or 8 carbon atoms.

3. Process for the preparation of an acetylacetoxy-alkyl-allyl ether according to claim 1 characterised in that a hydroxyalkyl-allyl ether having 2 to 10 carbon atoms in the alkyl group and 3 to 7 carbon atoms in the allyl group is reacted under common conditions with diketene.

4. Process for the preparation of an acetylacetoxyalkylallyl ether, characterised in that under common conditions at first an alcohol of formula (V)

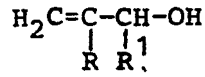

in which R and $R^1$ are defined as in formula (I), is reacted with an epoxide of formula (VI)

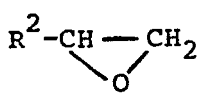

in which $R^2$ is defined as in formula (I), and thereafter the resulting hydroxyalkyl-allyl ether is reacted with diketene.

5. Process according to claim 4, characterised in that as alcohol of formula (V) there is used allyl alcohol or methallyl alcohol.

6. Process according to claim 4, characterised in that as epoxide there is used an epoxide of formula (VI), in which $R^2$ is a hydrogen atom, an alkyl group having 1, 2 or 3 carbon atoms which may be substituted by a halogen atom, a hydroxyl radical or an acyloxy radical having 3 to 6 carbon atoms, or an aryl group having 6, 7 and 8 carbon atoms.

7. Process according to claim 4, characterised in that the reactions are carried out in the presence of a catalyst.

8. Use of an acetylacetoxy-alkyl-allyl ether according to claim 1 as comonomer in the polymerisation of vinyl compounds.

**Claims** (for the contracting State AT)

1. Process for the preparation of an acetylacetoxy-alkyl-allyl ether, characterised in that a hydroxyalkyl-allyl ether having 2 to 10 carbon atoms in the alkyl group and 3 to 7 carbon atoms in the allyl group is reacted under common conditions, with diketene to yield a compound of formula (I)

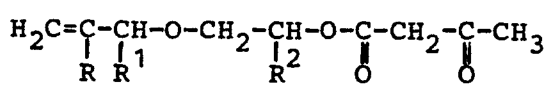

or of formula (II)

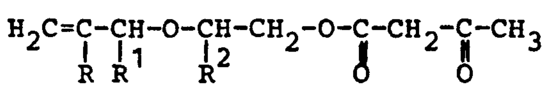

or mixtures thereof, in which formulae R is a hydrogen atom or a methyl group, $R^1$ is a hydrogen atom or an alkyl group having 1, 2 or 3 carbon atoms and $R^2$ is a hydrogen atom or a hydrocarbon radical having from 1 to 8 carbon atoms and optionally containing one or more oxygen atoms or a halogen atom.

2. Process for the preparation of an acetylacetoxy-alkyl-allyl ether, characterised in that, under common conditions, there is reacted first an alcohol of formula (V)

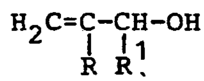

in which R is a hydrogen atom or a methyl group and $R^1$ is a hydrogen atom or an alkyl group having 1, 2 or 3 carbon atoms, with an epoxide of formula (VI)

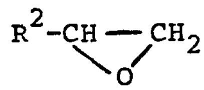

in which $R^2$ a hydrogen atom or a hydrocarbon radical having from 1 to 8 carbon atoms and optionally containing one or more oxygen atoms or a halogen atom.

3. Process according to claim 2, characterised in that as alcohol of formula (V) there is used allyl alcohol or methallyl alcohol.

4. Process according to claim 2, characterised in that as epoxide there is used an epoxide of formula (VI), in which $R^2$ is a hydrogen atom, an alkyl group having 1, 2 or 3 carbon atoms which may be substituted by a halogen atom, a hydroxyl radical or an acyloxy radical having 3 to 6 carbon atoms, or an aryl group having 6, 7 or 8 carbon atoms.

5. Process according to claim 2, characterised in that the reactions are carried out in the presence of a catalyst.

6. Process for the preparation of an unsaturated acetoacetic acid ester, characterised in that

a) a Williamson synthesis is carried out with a — optionally substituted — (2-hydroxyethyl)-acetoacetic acid ester and a — optionally substituted — allyl halide, or

b) a Williamson synthesis is carried out with a — optionally substituted — (2-halogenoethyl)-acetoacetic acid ester and a — optionally substituted — alkali metal allyl alcoholate, or

c) a — optionally substituted — allyl glycole ether is transformed in an allyl glycole acetic acid ester and the latter is reacted with acetoacetic acid ethyl ester by way of a Claisen condensation whereby an acetylacetoxyalkyl-allyl ether of formula (I)

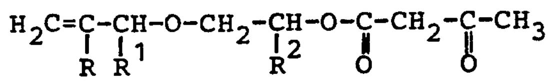

or of formula (II)

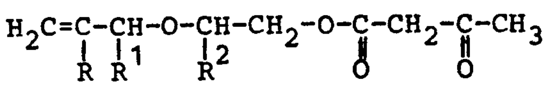

or mixtures thereof are obtained, in which formulae R is a hydrogen atom or a methyl group, $R^1$ is a hydrogen atom or an alkyl group having 1, 2 or 3 carbon atoms and $R^2$ is a hydrogen atom or a hydrocarbon radical having from 1 to 8 carbon atoms and optionally containing one or more oxygen atoms or a halogen atom.

7. Use of an acetylacetoxy-alkyl-allyl ether obtainable according to claim 1 or 6 as comonomer in the polymerisation of vinyl compounds.

**Revendications** (pour les Etats contractants : BE, CH, LI, DE, FR, GB, IT, NL, SE)

1. Oxydes d'acétylacétoxyalkyles et d'allyles répondant à la formule I

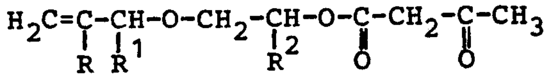

ou à la formule II

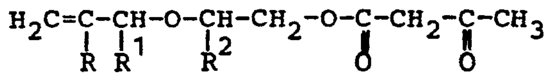

et leurs mélanges,
formules dans lesquelles R représente un atome d'hydrogène ou un radical méthyle, $R^1$ un atome d'hydrogène ou un radical alkyle contenant 1, 2 ou 3 atomes de carbone, et $R^2$ un atome d'hydrogène ou un radical hydrocarboné renfermant de 1 à 8 atomes de carbone, radical qui peut éventuellement contenir un ou plusieurs atomes d'oxygène ou un atome d'halogène.

2. Oxydes d'acétylacétoxyalkyles et d'allyles répondant à la formule III

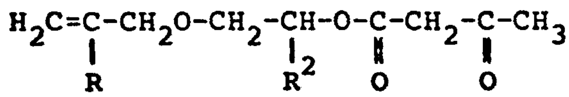

ou à la formule IV

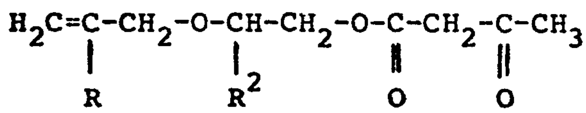

et leurs mélanges,
formules dans lesquelles R représente un atome d'hydrogène ou un radical méthyle et $R^2$ représente (a) un atome d'hydrogène, (b) un radical alkyle contenant 1, 2 ou 3 atomes de carbone, lequel peut porter un atome d'halogène, un groupe hydroxy, un radical acyloxy contenant de 3 à 6 atomes de carbone ou un radical acétylacétoxy, ou (c) un radical aryle contenant 6, 7 ou 8 atomes de carbone.

3. Procédé de préparation d'un oxyde d'acétyl-acétoxyalkyle et d'allyle selon la revendication 1, procédé caractérisé en ce qu'on fait réagir un oxyde d'hydroxy-alkyle et d'allyle contenant de 2 à 10 atomes de carbone dans le radical alkyle et de 3 à 7 atomes de carbone dans le radical allyle, dans les conditions habituelles, avec le dicétène.

4. Procédé de préparation d'un oxyde d'acétylacétoxyalkyle et d'allyle, procédé caractérisé en ce qu'on fait réagir d'abord un alcool répondant à la formule V

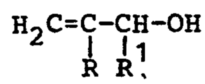

dans laquelle R et $R^1$ ont les significations données à propos de la formule I, avec un époxyde répondant à la formule VI

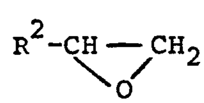

dans laquelle $R^2$ a la signification donnée à propos de la formule I, puis on fait réagir l'oxyde d'hydroxy-alkyle et d'allyle obtenu avec le décétène, cela dans les conditions habituelles.

5. Procédé selon la revendication 4, caractérisé en ce qu'on utilise, comme alcool de formule V, l'alcool allylique ou l'alcool méthallylique.

6. Procédé selon la revendication 4, caractérisé en ce qu'on utilise, comme époxyde, un époxyde de formule VI dans lequel $R^2$ représente un atome d'hydrogène, un radical alkyle contenant 1, 2 ou 3 atomes de carbone, éventuellement porteur d'un atome d'halogène, d'un groupe hydroxy ou d'un radical acyloxy contenant de 3 à 6 atomes de carbone, ou un radical aryle contenant 6, 7 ou 8 atomes de carbone.

7. Procédé selon la revendication 4, caractérisé en ce qu'on effectue les réactions en présence d'un catalyseur.

8. Application d'un oxyde d'acétylacétoxyalkyle et d'allyle selon la revendication 1 comme comonomère dans la polymérisation de composés vinyliques.

**Revendications** (pour l'Etat contractant AT)

1. Procédé de préparation d'un oxyde d'acétylacétoxyalkyle et d'allyle, procédé caractérisé en ce qu'on fait réagir avec le décétène, dans les conditions habituelles, un oxyde d'hydroxyalkyle et d'allyle

contenant de 2 à 10 atomes de carbone dans le radical alkyle et de 3 à 7 atomes de carbone dans le radical allyle, de manière à obtenir un composé répondant à la formule I

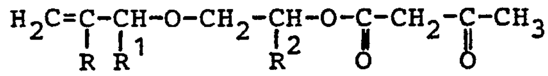

ou à la formule II

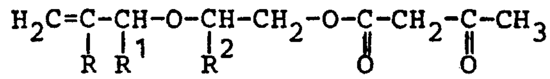

ou un mélange de composés de formule I et de formule II, formules dans lesquelles R représente un atome d'hydrogène ou un radical méthyle, $R^1$ représente un atome d'hydrogène ou un radical alkyle contenant 1, 2 ou 3 atomes de carbone, et $R^2$ représente un atome d'hydrogène ou un radical hydrocarboné renfermant de 1 à 8 atomes de carbone, lequel contient éventuellement 1 ou plusieurs atomes d'oxygène ou un atome d'halogène.

2. Procédé de préparation d'un oxyde d'acétylacétoxyalkyle et d'allyle, procédé caractérisé en ce qu'on fait d'abord réagir un alcool répondant à la formule V

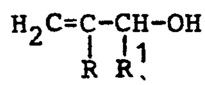

dans laquelle R représente un atome d'hydrogène ou un radical méthyle et $R^1$ un atome d'hydrogène ou un radical alkyle contenant 1, 2 ou 3 atomes de carbone, avec un époxyde répondant à la formule VI

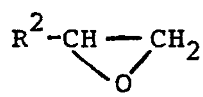

dans laquelle $R^2$ représente un atome d'hydrogène ou un radical hydrocarboné en $C_1 - C_8$ éventuellement porteur d'un ou plusieurs atomes d'oxygène ou d'un atome d'halogène, puis on fait réagir avec le dicétène l'oxyde d'hydroxyalkyle et d'allyle obtenu, cela dans les conditions habituelles.

3. Procédé selon la revendication 2, caractérisé en ce qu'on utilise, comme alcool de formule V, l'alcool allylique ou l'alcool méthallylique.

4. Procédé selon la revendication 2, caractérisé en ce qu'on utilise, comme époxyde, un époxyde de formule VI dans lequel $R^2$ représente un atome d'hydrogène, un radical alkyle contenant 1, 2 ou 3 atomes de carbone, éventuellement porteur d'un atome d'halogène, d'un groupe hydroxy ou d'un radical acyloxy contenant de 3 à 6 atomes de carbone, ou un radical aryle contenant 6, 7 ou 8 atomes de carbone.

5. Procédé selon la revendication 2, caractérisé en ce qu'on effectue les réactions en présence d'un catalyseur.

6. Procédé de préparation d'un ester acétylacétique insaturé, procédé caractérisé en ce que :

a) on effectue une synthèse de Williamson avec un sel de métal alcalin d'un acétylacétate d'hydroxy-2 éthyle, éventuellement substitué, et un halogénure d'allyle, éventuellement substitué, ou

b) on effectue une synthèse de Williamson avec un acétylacétate d'halogéno-2 éthyle, éventuellement substitué, et un allylalcoolate de métal alcalin, éventuellement substitué, ou

c) on transforme un éther allylique de glycol, éventuellement substitué, en un acétate d'allyl-glycol, et on fait réagir ce dernier, par une condensation de Claisen, avec l'acétylacétate d'éthyle,

d'où obtention d'un oxyde d'acétylacétoxyalkyle et d'allyle répondant à la formule I

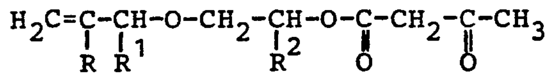

ou à la formule II

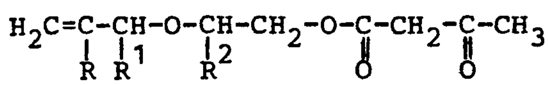

ou de leurs mélanges,

formules dans lesquelles R représente un atome d'hydrogène ou un radical méthyle, $R^1$ représente un atome d'hydrogène ou un radical alkyle contenant 1, 2 ou 3 atomes de carbone, et $R^2$ représente un

atome d'hydrogène ou un radical hydrocarboné renfermant de 1 à 8 atomes de carbone, lequel contient éventuellement un ou plusieurs atomes d'oxygène ou un atome d'halogène.

7. Application d'un oxyde d'acétylacétoxy-alkyle et d'allyle pouvant être préparé selon la revendication 1 ou la revendication 6 comme comonomère dans la polymérisation de composés vinyliques.